# EUROPEAN PATENT SPECIFICATION

(11) **EP 1 233 057 B1**
(45) Date of publication and mention of the grant of the patent: **04.03.2015**
(21) Application number: 00974940.9
(22) Date of filing: 10.11.2000
(51) Int. Cl.: C12N 1/20, C12N 1/00, C12N 9/00, C12N 11/16

(54) **STERILIZED MICROBIAL CELLS**
STERILISIERTE, MIKROBIELLE ZELLEN
CELLULES MICROBIENNES STERILISEES

(30) Priority: 12.11.1999 JP 32283699
(43) Date of publication of application: 21.08.2002
(73) Proprietor: MITSUBISHI RAYON CO., LTD., Tokyo 100-8253 (JP)
(72) Inventor: WATANABE, Fumiaki, c/o Chemicals Development Laboratories, Yokohama-shi Kanagawa 230-0053 (JP); OKAZAKI, Hanako, c/o Chemicals Development Laboratories, Yokohama-shi Kanagawa 230-0053 (JP); ABE, Yasuteru, c/o Tokyo Technology And Information Center, Kawasaki-shi, Kanagawa 214-0014 (JP); MIZUNASHI, Wataru, c/o Chemicals Development Laboratories, Yokohama-shi Kanagawa 230-0053 (JP); KANOU, Makoto, c/o Chemicals Development Laboratories, Yokohama-shi Kanagawa 230-0053 (JP); MURAO, Kouzo, c/o Chemicals Development Laboratories, Yokohama-shi Kanagawa 230-0053 (JP)
(74) Representative: Hoffmann Eitle
(86) International application number: PCT/JP2000/007969
(87) International publication number: WO 2001/036592

(56) References cited:
- EP-A1- 0 338 439
- BE-A- 858 992
- GB-A- 1 174 045
- JP-A- 2 055 794
- JP-A- 9 052 804
- JP-A- 52 003 833
- JP-A- 61 152 276
- US-A- 3 282 775
- US-A- 4 925 801
- US-A- 5 863 547
- US-A- 5 976 462
- DATABASE WPI Section Ch, Week 197708 Derwent Publications Ltd., London, GB; Class A97, AN 1977-13719Y XP002295415 & JP 52 003833 A (UENO M) 12 January 1977 (1977-01-12)
- DATABASE WPI Section Ch, Week 198634 Derwent Publications Ltd., London, GB; Class B04, AN 1986-221824 XP002295416 & JP 61 152276 A (SUNTORY LTD) 10 July 1986 (1986-07-10)
- DATABASE WPI Section Ch, Week 199718 Derwent Publications Ltd., London, GB; Class C02, AN 1997-197180 XP002295417 & JP 09 052804 A (TAKEDA CHEM IND LTD) 25 February 1997 (1997-02-25)
- DATABASE WPI Section Ch, Week 199014 Derwent Publications Ltd., London, GB; Class D22, AN 1990-104332 XP002295418 & JP 02 055794 A (ASAHI CHEM IND CO LTD) 26 February 1990 (1990-02-26)
- ROSA PALLANZA ET AL.: 'In vitro activity of A-16686, a potential antiplaque agent' ANTIMICROBIAL AGENTS AND CHEMOTHERAPY vol. 26, no. 4, 1984, pages 462 - 465, XP002936587
- DATABASE WPI Week 198634, Derwent Publications Ltd., London, GB; AN 1986-221824, XP002295416 SUNTORY LTD & JP S61 152 276 A 10 July 1986

## Description

### TECHNICAL FIELD

The present invention relates to a process of sterilising a bacterium as defined in the main claim.

### BACKGROUND ART

In the industrial use of microbial cells, there is anxiety that a secondary microbial contamination possibly occurs due to leaking while manufacturing, mixing in a product, or mishandling after the use. Usually, a reaction is carried out in a closed system using highly safe microbial cells, and the microbial cells are removed from the reactant solution by filtration with an auxiliary agent such as diatomaceous earth. Alternatively, it is general to take a measure for immobilizing the microbial cells so as to minimize the leak of the microbial cells. In this case, with a view to using the highly safe microbial cells, importance was not placed on a complete sterilization of microbial cells. However, while attention is given to manufacturer's liability in recent years, it is required to prevent the leak of viable cells even in the case of using highly safe microbial cells.

Further, in the case of using recombinant cells, complete sterilization of microbial cells is required for handling them in a system except a closed one in accordance with various guidelines. Examples of common sterilization methods include heat treatment under a condition which does not deactivate the enzyme of interest, or use of a crude enzyme solution obtained by disrupting cells. As a sterilization method by using a drug or the like, a surfactant is generally used. Sterilization is achieved by disruption of cell membranes of microbial cells by the surfactant, denaturation of an enzyme protein important for life support, or the like. In particularly, use of a cationic surfactant or an ampholytic surfactant exhibits a high sterilization effect.

As the prior art, there is disclosed a method for sterilizing a recombinant *E*. *coli* by adding benzalkonium chloride. (refer to Japanese Patent Application Laying-Open No. 61-152276)

When heat treatment is employed as a sterilization method, the enzyme of interest or the like should be heat-resistant. Otherwise, it cannot be employed. When a crude enzyme solution is prepared by disrupting cells, complicated operations should be conducted for preparing the crude enzyme solution. Additionally, separation procedure of the enzyme from the reactant solution becomes much heavier burden in comparison with the case wherein cells are used therewith. Thus, there are many drawbacks in view of industrial use.

Although a sterilization method by using an ionic surfactant is known, in general it has a bacteria elimination effect by sterilizing a low concentration of microorganisms, and the achievement of sterilization of a high concentration of microbial cells, what is disclosed in the present invention, has not completely been known.

The prior art (Japanese Patent Application Laying-Open No. 61-152276) is a method applied only for *E. coli.* Then, because this is employed in extracting and purifying a physiologically active substance, disruption and agglutination of cells are not a demerit. However, the sterilized microbial cells obtained by the present invention as it is, or after immobilization, are used as a reactive catalyst for material production. Thus, disruption or agglutination of cells becomes a cause for reduction of enzyme activity or reactivity, and trouble of immobilization, and they are extremely disadvantageous for industrial use.

GB 1,174,045 describes a fermentation process for the production of an enzyme preparation capable of enzymatically hydrolyzing penicillins. In an exemplary embodiment cetyltrimethylammonium bromide is added to a fermentation broth comprising E. coli.

US 3 282 775 describes a chemical sterilization composition comprising combinations of saturated dialdehydes and cationic surface active agents, which have broad spectrums of sporicidaal activity and an extremely fast rate of killing certain bacterial spores.

US 4 925 801 describes a process for preserving the phosphorylating activity of yeast. After glutaraldehyde treatment the yeast is added to nutrient mixture for incubation. It is taught that the cells can be kept in a nutrient mixture and show metabolic activity cells for at least one week.

### DISCLOSURE OF THE INVENTION

The inventors made studies on a method for sterilizing, under a relatively moderate condition, viable microbial cells having produced therein a highly versatile and industrially useful enzyme without deactivation of the enzyme. They found sterilization of microbial cells could be accomplished by treating the microbial cells in the presence of a surfactant without deactivation of an industrially useful enzyme, and thereby completed the present invention. In the present invention, "sterilization of microbial cells" means that the number of viable cells is set at substantially zero in a relatively high concentration of microbial cell suspension. Herein, the number of viable cells is set at substantially zero means that the survival rate of the microbial cells becomes 1/10⁻⁵ or less. The survival rate is represented by the rate of the number of viable cells in microbial cell suspension solution to the number of viable cells still existing in sterilization-treated microbial cell suspension solution. The number of viable cells is calculated as follows; diluting a microbial cell suspension solution as a measurement object to the predetermined concentration: inoculating the microbial cell suspension solution onto a viable solid medium; and computing from the dilution factor and the number of microbial cells grown on the solid medium.

Namely, the present invention provides
1. A process for the sterilisation of a bacterium without deactivation of enzymatic activity, comprising the addition of
   (i) a surfactant
   (ii) glutaraldehyde
      wherein the surfactant and glutaraldehyde are added to the bacterium so as to have concentrations of 0.05 to 10% and 0.05 to 5%, respectively, and
      wherein the bacterium has a dry cell weight of 5 to 200 g/L in an aqueous medium,
      wherein the bacterium has at least one enzyme activity selected from the group consisting of nitrile hydratase, nitrilase, amidase, fumarase, aspartase, and ethylenediamine-N, N'-disuccinic acid: ethylenediamine lyase; and
      wherein the surfactant is a cationic surfactant or an ampholytic surfactant and wherein the bacterium is selected from a gene-recombinant bacterium, Rhodococcus rhodochrous having the deposit number FERM BP-1478, Rhodococcus rhodochrous having the deposit number FERM BP-3733, Rhodococcus sp. having the deposit number FERM BP-3324, Rhodococcus sp. having the deposit number FERM-BP-6231, Bacillus subtilis having the deposit number ATCC 6051, Bacillus stearothermophilus having the deposit number ATCC 12016, Rhodococcus rhodochrous having the deposit number ATCC 12674, Rhodococcus rhodochrous ATCC 12674/pAR016 as described in JP 10-337185, and Rhodococcus rhodochrous having the deposit number FERM BP-6548.
2. The process according to item 1, wherein the cationic surfactant is at least one member selected from the group consisting of benzethonium chloride, cetylpyridinium chloride, methylstearoyl chloride, and cetyltrymethylammonium bromide.
3. The process according to item 1, wherein the bacterium is a gene recombinant bacterium.
4. The process according to item 3, wherein the gene recombinant bacterium is a genus Rhodococcus bacterium.
5. A solution containing a sterilised, bacterium, wherein the bacterium has a dried cell weight of 5 to 200 g/L in an aqueous medium, comprising
   (i) a surfactant having a concentration of 0.05 to 10%,
   (ii) glutaraldehyde having a concentration of 0.05 to 5%,
      wherein the bacterium has at least one enzyme activity selected from the group consisting of nitrile hydratase, nitrilase, amidase, fumarase, aspartase, and ethylenediamine-N, N'disuccinic acid, ethylenediamine lyase, wherein the enzymatic activity is not deactivated, and wherein the surfactant is a cationic surfactant or an ampholytic surfactant and wherein the bacterium is selected from a gene-recombinant bacterium, Rhodococcus rhodochrous having the deposit number FERM BP-1478, Rhodococcus rhodochrous having the deposit number FERM BP-3733, Rhodococcus sp. having the deposit number FERM BP-3324, Rhodococcus sp. having the deposit number FERM-BP-6231, Bacillus subtilis having the deposit number ATCC 6051, Bacillus stearothermophilus having the deposit number ATCC 12016, Rhodococcus rhodochrous having the deposit number ATCC 12674, Rhodococcus rhodochrous ATCC 12674/pAR016 as described in JP 10-337185, and Rhodococcus rhodochrous having the deposit number FERM BP-6548.
6. The solution according to item 5, wherein the cationic surfactant is at least one member selected from the group consisting of benzethonium chloride, cetylpyridinium chloride, methylstearoyl chloride, and cetyltrymethylammonium bromide.
7. The solution according to item 6, wherein the concentration of benzethonium chloride is 0.05 to 5%.
8. The solution according to item 5, wherein the gene-recombinant bacterium is a genus Rhodococcus bacterium.

The microbial catalyst of interest according to the present invention is a microbial cell to be used as a biocatalyst for producing chemicals as listed below or a gene-recombinant bacterium. Examples of the microbial cell include *Rhodococcus rhodochrous* J-1 producing nitrile hydratase which converts acrylic nitrile to acrylamide, recombinant *R. rhodochrous* ATCC12674/pKNH2 introducing a nitrile hydratase gene, *Rhodococcus sp.* SK92 producing nitrilase which converts acrylic nitrile to acrylic acid, *Rhodococcus sp.* EA4 producing amidase which converts glycinamide to glycine, *Bacillus subtilis* ATCC 6051, *Bacillus stearothermophilus* ATCC 12016 and *Rhodococcus rhodochrous* ATCC 12674 producing fumarase which converts fumaric acid to malic acid, recombinant *Rhodococcus rhodochrous* ATCC 12674/pAR016 introducing a gene for aspartase which converts fumaric acid and ammonium to L- asparatic acid, and recombinant *Rhodococcus rhodochrous* ATCC 17895/pSE001 containing a gene for an enzyme which converts fumaric acid and diamines to polyamino carboxylic acids.

The above *Rhodococcus rhodochrous* J-1 was deposited under the terms of the Budapest Treaty with the National Institute of Bioscience and Human-Technology, Agency of Industrial Science and Technology, Ministry of Internatioanl Trade and Industry (1-3, Higashi 1-chome, Tsukuba, Ibaraki, Japan) under accession No. FERM BP-1478 on September 18, 1987. The above *R. rhodochrous* ATCC 12674/pKNH2 was deposited under the terms of the Budapest Treaty with the National Institute of Bioscience and Human-Technology, Agency of Industrial Science and Technology, Ministry of Internatioanl Trade and Industry (1-3, Higashi 1-chome, Tsukuba, Ibaraki, Japan) under accession No. FERM BP-3733 on March 1, 1991. The above *Rhodococcus sp.* SK92 was deposited under the terms of the Budapest Treaty with the National Institute of Bioscience and Human-Technology, Agency of Industrial Science and Technology, Ministry of Internatioanl Trade and Industry (1-3, Higashi 1-chome, Tsukuba, Ibaraki, Japan) under accession No. FERM BP-3324 on February 21, 1990. The *Rhodococcus sp.* EA4 was deposited under the terms of the Budapest Treaty with the National Institute of Bioscience and Human-Technology, Agency of Industrial Science and Technology, Ministry of Internatioan1 Trade and Industry (1-3, Higashi 1-chome, Tsukuba, Ibaraki, Japan) under accession No. FERM BP-6231 on March 28, 1991. The above *Rhodococcus rhodochrous* ATCC17895/pSE001 was deposited under the terms of the Budapest Treaty with the National Institute of Bioscience and Human-Technology, Agency of Industrial Science and Technology, Ministry of Internatioanl Trade and Industry (1-3, Higashi 1-chome, Tsukuba, Ibaraki, Japan) under accession No. FERM BP-6548 on September 18, 1997.

Further, with respect to the recombinant microorganisms whose accession numbers are not described herein, the preparation methods therefor are indicated below. The other microorganisms except for them are known, and they are easily available from American Type Culture Collection (ATCC). The recombinant *Rhodococcus rhodochrous* ATCC 12674/pAR016 which is used for producing L- asparatic acid from fumaric acid and ammonium to was prepared by the method described in Japanese Patent Application Laying-Open No. 10-337185.

The recombinant *Rhodococcus rhodochrous* ATCC 17895/pSE001 (FERM BP-6548) which is used for producing polyamino carboxylic acids from fumaric acid and diamines, was prepared as follows.

To 2 µl of the plasmid pED020 (described in Japanese Patent Application Laying-Open No. 10-210984), 2 µl of a 10 × restriction enzyme buffer solution, 15 µl of sterilized water and 1 µl of restriction enzyme XhoI were added and the mixture was reacted at 37°C for 2 hours. The plasmid was collected by ethanol precipitation, and after being dried, 15 µl of sterilized water, 2 µl of a 10 × buffer solution for Klenow fragment, 2 µl of 10 mM dNTP solution, and 1 µl of Klenow fragment were added thereto and the mixture was reacted at 37°C for 2 hours. By ethanol precipitation a DNA fragment was collected, and after being dried 8 µl of sterilized water, 1 µl of XbaI of linker solution, 16 µl of solution A of Ligation kit (available from TaKaRa Shuzo Co.; Ltd.) and 4 µl of solution B of the Ligation kit were added thereto and the mixture was reacted at 16°C for 4 hours. Thereafter, the DNA fragment was transformed to JM109. From the thus obtained recombinant, prepared was a plasmid wherein an Xhol site of pED020 was converted to an XbaI site. To 2 µl of the obtained plasmid, 2 µl of a 10 × restriction enzyme buffer solution, 15 µl of sterilized water, and 1 µl of a restriction enzyme EcoRV were added thereto, and the mixture was reacted at 37°C for 2 hours. By ethanol precipitation a DNA fragment was collected and after being dried 8 µl of sterilized water, 1 µl of Sse8387I linker solution, 16 µl of solution A of Ligation kit (available from TaKaRa Shuzo Co., Ltd.) and 4 µl of solution B of the Ligation kit were added thereto and the mixture was reacted at 16°C for 4 hour. Thereafter, the DNA fragment was transformed to JM109. From the thus obtained recombinant, prepared was a plasmid wherein an EcoRv site was converted to an Sse8387I site. To 2 µl of the obtained plasmid, 2 µl of 10 × restriction enzyme buffer solution, 14 µl of sterilized water, 1 µl of restriction enzyme XbaI and 1 µl of Sse8387I were added thereto, and the mixture was reacted at 37°C for 2 hours. Then, the resulting mixture was separated by 0.7 % agarose gel electrophoresis, thereby collecting 1.7 Kb of band. The band was inserted into XbaI-Sse8387I site of the a plasmid pSJ034 containing a promoter for *Rhodococcus* bacteria, thereby preparing a recombinant plasmid pSE001.

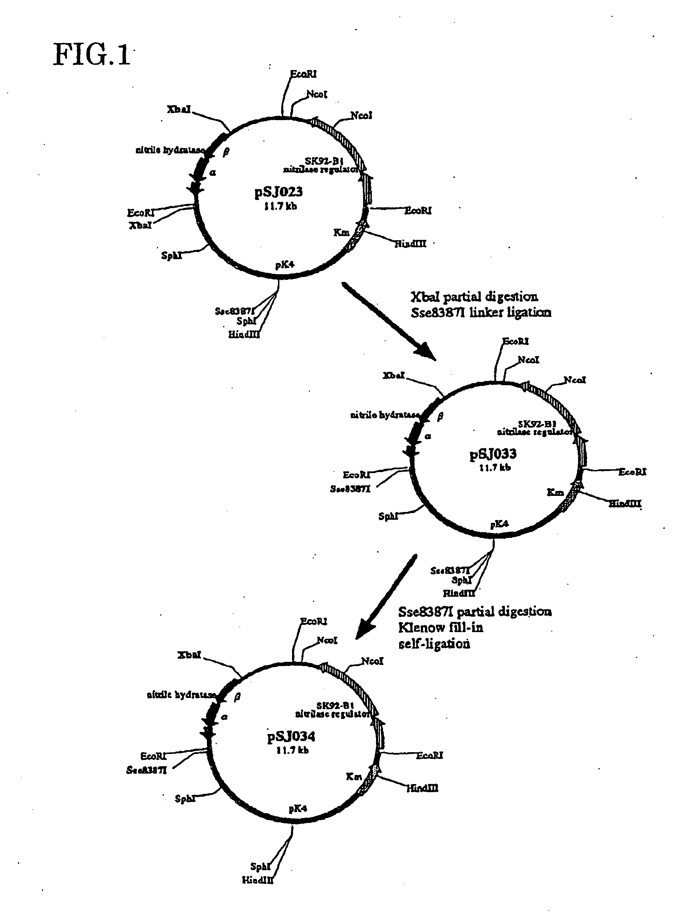

A pSJ034 was prepared with a plasmid pSJ023 (described in Japanese Patent Application No. 9-65618) by a process shown in Fig. 1.

Cells of *Rhodococcus rhodochrous* ATCC17895 strain were collected at mid-log phase by centrifugation, washed three times with ice-cold sterilized water, and then suspended in sterilized water. 1 µl of plasmid pSE001 and 10 µl of the cell suspension were mixed and ice-colded. The cell suspension and DNA were poured into a cuvette, electric pulse treatment was carried out by a gene transfer device Gene Pulser (BIO RAD) at 2.0 KV and 200 OHMS. The electric-pulse-treated solution was left to stand with ice-colded for 10 minutes, and then heat shock was given to the solution at 37°C for 10 minutes. 500 µl of MYK medium (containing 5 g/L polypepton, 3 g/L bacto-yeast extract, 3 g/L bact-malt extract, 2 g/L K₂HPO₄, and 2 g/L KH₂PO₄) was added thereto and the mixture was left to stand at 30°C for 5 hours. Then, the mixture applied onto MYK agar medium containing 50 mg/L kanamycin, and cultured at 30°C for 3 days, thereby preparing recombinant *Rhodococcus rhodochrous* ATCC17895/pSE001 (FERM BP-6548).

In the present invention, the sterilization treatment means that a surfactant and glutaraldehyde are applied to a microbial cell contained in a microbial catalyst as defined in claim 1. Specifically, the sterilization treatment is a treatment wherein the surfactant and, if necessary other additive are dissolved into the microbial cell suspension for contacting the microbial cell with the surfactant or with the surfactant and the additive. It is preferable to conduct the sterilization treatment with keeping cold or warm. By conducting the sterilization treatment at the predetermined temperature, it is possible to keep the capability of microbial catalyst high. Further, the sterilization treatment may be conducted with shaking.

In treating microbial cells, there may be used a culture solution, microbial cells prepared by washing the culture solution with a suitable buffer solution or the like, or treated substance thereof. Additionally, in the sterilization treatment, it is preferable to conduct, for example, drug-treatment, cell disruption treatment or the like in advance for the purpose of enhancing washability, cell membrane permeability, and microbial cell stability or the like.

The concentration of the microbial cells in sterilization treatment is not less than the cell concentration of the culture solution. In view of industrial use, the cell concentration in sterilization treatment is 5 to 200 g/L dry cell weight According to the present invention reliable sterilization treatment can be performed on a high concentration of microbial cell suspension. However, when the cell concentration exceeds 200 g/L dry cell weight, though depending on the kind and property of a microorganism, in general the flowability of the cell suspension becomes remarkably low, which deteriorates the dispersibility and solubility of the surfactant or the like. This leads to the fear of hampering efficient treatment for sterilization of microbial cells. Therefore, the cell concentration in sterilization treatment is from 5 to 200 g/L.

The treatment temperature in sterilization treatment of microbial cells may be from freezing temperature to 80°C, preferably 0 to 70°C. This treatment temperature is not particularly limited, but it may arbitrarily be set in accordance with the stability of the enzyme of interest.

The surfactant to be added in sterilization treatment of microbial cells is a cationic surfactant, or an ampholytic surfactant. Examples of the cationic surfactant include benzethonium chloride, cetylpyridinium chloride, methylstearoyl chloride, and cetyltrimethylammonium bromide. Examples of the ampholytic surfactant include carboxy betaine and aminocarboxylate. It is known that the cationic surfactant and the ampholytic surfactant are excellent in sterilization effect. Since a low concentration thereof can effectively sterilize microbial cells, they are used in the invention. Further, for sterilization treatment of the microbial cells, these surfactants may be used alone or, if necessary mixed in combination of two or more thereof.

For sterilization treatment of the microbial cells, the concentration of the surfactant is 0.05 to 10 %. When the concentration of the surfactant is too low, a sufficient effect cannot be expected for sterilizing the microbial cells. On the other hand, too high concentration thereof will cause problems such as handling of waste fluid after the treatment, deactivation of an industrially useful enzyme, or the like, and thus it is desirable to determine an optimum concentration with consideration given to microbial cells to be used and the stability of enzyme of interest.

Moreover, in the case of using an ionic surfactant, addition of a high concentration of the solution may cause bacteriolysis, disruption, and agglutination of microbical cells in sterilization treatment. It is effective to add the surfactant at a low concentration. The concentration thereof may be 0.5 to 10%, preferably about 1 to 8 %.

In the sterilization treatment, the treatment solution may have a pH of 4 to 11, preferably 5 to 10. It is desired to determine a pH value for the treatment with consideration given to the stability of enzyme of interest or the like.

In the sterilization treatment, it is also effective to add various additives for the purpose of enhancing efficacy of the treatment. Examples of the additives include alcohols such as ethanol, thiols such as 2-mercaptoethanol, amines such as ethylenediamine, amino acids such as cysteine, ornithine, and citrulline, and aldehydes such as glutaraldehyde.

Further, for the purpose of enhancing the stability of the enzyme of interest, it is effective to add a substrate, a product, an inhibitor or the like of the enzyme of interest. Examples thereof include various nitriles, various amides, various organic acids, amino acids, and polyamino carboxylic acid. With consideration given to the stability of the enzyme of interest, these additives may desirably be used alone or mixed in an optimum combination of two or more thereof, and an optimum concentration of these additives is desired to be determined.

### BRIEF DESCRIPTION OF THE DRAWING

Fig. 1 is a view illustrating a method for producing a plasmid pSJ034.

### BEST MODE FOR CARRYING OUT THE INVENTION

Hereinbelow, the present invention will be described in greater detail with reference to the following Examples. However, these Examples should not be construed as limiting the technical scope of the invention.

### Example 1

### Rhodococcus rhodochrous ATCC 17895/pSE001 (FERM BP-6548)

### 1. Cultivation

*Rhodococcus rhodochrous* ATCC 17895/pSE001 (FERM BP-6548) was inoclurated into 200 ml of MYK-Km medium (MYK culture medium contains 50 mg/L of kanamycin), and cultured at 30 °C for 72 hours. The resulting culture was, by using a 30 L jar, added to 20 L of a culture medium (pH 7.2) comprising 15 g/L of glucose, 10 g/L of sodium glutamate, 1 g/L of bactoyeast extract, 0.5 g/L of KH₂PO₄, 0.5 g/L of K₂HPO₄, 0.5 g/L of magnesium sulfate, and 50 mg/L of kanamycin, and subjected to aerobic cultivation with shaking at 30 °C for 60 hours. Then, 20 L of the obtained culture solution was circulated in a hollow fiber membrane clothflow SF FILTER KL-F-8102 (Kuraray Co., Ltd.) by a mohno pump (1 m/s) so as to be condensed. After discharging 10 L of the culture filtrate, whenever 2 L of the culture filtrate were discharged, 2 L of 100 mM boric acid buffer solution (pH 9.2) was added thereto, and a continuous washing was conducted by use of 20 L of the buffer solution in total, thereby preparing 10 kg of the washed cells. The washed cells obtained had a cell concentration of 42 g/L in terms of dry cell weight.

### 2. Sterilization treatment

To 50 ml of the washed cells, 1 ml of each 5 % surfactant solution as shown in Table 1 was added, and the treatment was conducted at 45 °C.

### 3. Determination of viable cell count

After the cell treatment was finished, the cells were twice washed with the same volume of 100 mM boric acid buffer solution (pH 9.2) as the washed cells to be used, and suspended so as to be the same volume as the washed cells. 0.1 ml of each suspension was applied onto MYK-Km agar medium, and subjected to cultivation at 30 °C for 3 days. Then, viable cell count was determined.

### 4. Determination of enzyme activity

1 ml of each suspension containing sterilized cells was suspended in 50 ml of an aqueous solution at pH 8.0 containing 342 mM fumaric acid and 171 mM ethylenediamine, and subjected to reaction for 24 hours. After the cells were removed from the reaction solution by centrifugation, the amount of ethylenediamine-N, N'-disuccinic acid in the obtained supernatant was analized by HPLC, thereby checking the activity of ethylenediamine-N, N'-disuccinic acid: ethylenediaminelyase (hereinafter abbreviated as EDDS-ase). (colum; WAKOSIL 5C8 (Wako Pure Chemical Industries, Ltd.), eluent; 50 mN phosphoric acid (pH 2.0) containing 10 mM tetra-n-butylammonium hydroxide and 0.4 mM CuSO₄,) The EDDS-ase activity of non-sterilized washed cells was taken as 100 for obtaining the remaining activity rate of EDDS-ase activity of the treated cells.

**Table 1**

| | Surfactant | Treatment time(h) | Viable cell count (cells/ml) | Remaining activity (%) |
|---|---|---|---|---|
| 1 | no | 8 | >10³ | 98 |
| 2 | no | 96 | >10² | 33 |
| 3 | KS602 | 72 | 0 | 56 |
| 4 | LG126 | 72 | 0 | 62 |
| 5 | PLURONIC L61 | 96 | 0 | 41 |
| 6 | EMULGEN109P | 48 | 0 | 84 |
| 7 | Toriton X-100 | 48 | 0 | 87 |
| 8 | cetylpyridinium chloride | 24 | 0 | 65 |
| 9 | methylstearoyl chloride | 24 | 0 | 64 |
| 10 | benzethonium chloride | 24 | 0 | 67 |

### Example 2

To 50 ml of the washed cells prepared by the method of Example 1, 2.5% benzethonium chloride solution was added so as to have the concentrations as shown in Table 2, and treatment was conducted at 10 °C or 30 °C for 8 hours. After the treatment, the viable cell count and the remining activity rate of EDDS-ase were obtained.

**Table 2**

| | Conc, of benzethonium chloride | Treatment Temp. (°C) | Viable cell count (cells/ml) | Remaining activity (%) |
|---|---|---|---|---|
| 1 | no | 10 | >10⁷ | 100 |
| 2 | no | 30 | >10⁶ | 100 |
| 3 | 0.1% | 10 | 125 | 98 |
| 4 | 0.1% | 30 | 10 | 74 |
| 5 | 0.25% | 10 | 0 | 96 |
| 6 | 0.25% | 30 | 0 | 57 |
| 7 | 0.5% | 10 | 0 | 94 |
| 8 | 0.5% | 30 | 0 | 12 |

### Example 3

To 50 ml of the washed cells prepared by the method of Example 1, 10 ml of 30 % ethylenediamine-N, N'-disuccinic acid solution (pH 8.5) containing 0.6 %, 1.5 %, and 3 % of benzethonium chloride were added so as to have the concentrations as shown in Table 3, and cell treatment was conducted at 30 °C or 50 °C for 3 hours. After the treatment, the viable cell count and the remining activity rate of EDDS-ase were obtained.

**Table 3**

| | Conc. of benzethonium chloride | Treatment Temp. (°C) | Viable cell count (cells/ml) | Remaining activity (%) |
|---|---|---|---|---|
| 1 | no | 30 | >10⁶ | 98 |
| 2 | no | 50 | >10³ | 106 |
| 3 | 0.1% | 30 | 142 | 92 |
| 4 | 0.1% | 50 | 10 | 98 |
| 5 | 0.25% | 30 | 0 | 89 |
| 6 | 0.25% | 50 | 0 | 98 |
| 7 | 0.5% | 30 | 0 | 91 |
| 8 | 0.5% | 50 | 0 | 0 |

### Example 4

To 50 ml of the culture solution prepared by the method of Example 1,2% surfactant solution of each in Table 4 was added so as to have the concentrations as shown in Table 4, and cell treatment was conducted at 4°C or 30°C for 3 hours. After the treatment, the viable cell count and the remining activity rate of EDDS-ase were obtained.

**Table 4**

| | Surfactant | Treatment Temp. (°C) | Viable cell count (cells/ml) | Remaining activity (%) |
|---|---|---|---|---|
| 1 | no | 4 | >10⁹ | 100 |
| 2 | no | 30 | >10⁶ | 68 |
| 3 | 0.1% cetylpyridinium chloride | 4 | 0 | 114 |
| 4 | 0.1% cetylpyridinium chloride | 30 | 0 | 106 |
| 5 | 0.05% cetylpyridinium chloride | 4 | >10² | 115 |
| 6 | 0.05% cetylpyridinium chloride | 30 | 0 | 100 |
| 7 | 0.1% methylstearoyl chloride | 4 | 0 | 120 |
| 8 | 0.1 % methylstearoyl chloride | 30 | 0 | 102 |
| 9 | 0.1 % benzethonium chloride | 4 | 0 | 98 |
| 10 | 0.1% benzethonium chloride | 30 | 0 | 96 |
| 11 | 0.05% benzethonium chloride | 4 | 0 | 102 |
| 12 | 0.05% benzethonium chloride | 30 | 0 | 103 |

### Example 5

### (1) Rhodococcus rhodochrous J-1 (FERM BP-1478)

### 1. Cultivation

Prepared was a medium (pH 7.2) comprising 10 g/L of glucose, 0.5 g/L of K₂HPO₄, 0.5 g/L of KH₂PO₄, 0.5 g/L of MgSO₄·7H₂O, 1 g/L of yeast extract, and 7.5 g/L of peptone. 100 ml of the medium was dispensed into a 500 ml Erlenmeyer flask and sterilized by an autoclave at 120 °C for 15 minutes. *R. rhodochrous* J-1 (FERM BP-1478) was inoculated into this medium and subjected to shaking cultivation at 28 °C for 3 days. I ml of the thus obtained culture solution was inoculated into the same medium (20 Erlenmeyer flasks), to which simultaneously 15 g/L of urea and 10 mg/L of CoCl₂ were added, and subjected to a shaking cultivation at 28 °C for 96 hours. Then, cells were collected by centrifugation, and the collected cells were washed with the same volume of 50 mM phosphoric acid buffer solution (pH 7.7) as the culture solution. Thereafter, the obtained cells were suspended in the 100 ml of the buffer solution, thereby preparing washed cells. The washed cells obtained had a cell concentration of 105 g/L in terms of dry cell weight.

### 2. Sterilization treatment

To the washed cells, benzethonium chloride solution and/or glutaraldehyde solution were added so as to have the concentrations as shown in Table 5, respectively, and sterilization treatment was conducted at 30 °C for 5 hours. Incidentally, for comparison, instead of 1.5% benzethonium chloride solution and/or glutaraldehyde solution, phosphoric acid buffer solution was added, and the same sterilization treatment was conducted.

### 3. Determination of viable cell count

After the treatment, the cells were washed three times with the buffer solution which had been used for the preparation of the washed cells, and suspended with the same buffer solution so that the volume became equal to one at sterilization treatment. Then, 0.1 ml of each suspension was applied on MYK agar medium and subjected to cultivation at 30 °C for 3 days, then obtaining viable cell count.

### 4. Determination of enzyme activity

With respect to nitrile hydratase activity, after 2 ml of the reaction mixture containing 1.0 ml of 1M acrylic nitrile, 0.5 mi of 0.1 M potassium phosphate buffer solution (pH 7.0) and the washed cells to have been subjected to sterilization treatment, were reacted at 10 °C for a predetermined period, 0.2 ml of IN HCl was added for stopping the reaction, and thereafter the amount of acrylamide produced was analyzed by gas chromatography for determination of the nitrile hydratase activity.

### (2) R. rhodochrous ATCC12674/pKNH2 (FERM BP-3733)

### 1. Cultivation

Prepared was a medium (pH 7.2) comprising 10 g/L of glycerol, 5 g/L of polypepton, 3 g/L of yeast extract, and 3 g/L of Malt extract. 100 ml of the medium was dispensed into a 500 ml Erlenmeyer flask and sterilized by an autoclave at 120 °C for 15 minutes. *R. rhodochrous* ATCC12674/pKNH2 (FERM BP-3733) was inoculated into this medium and subjected to a shaking cultivation at 25 °C for 2 days. 1 ml of the thus obtained culture solution was inoculated into the same medium (20 Erlenmeyer flasks), to which simultaneously isobutyronitrile and isobutyramide were added so as to each be 1 g/L, and subjected to a cultivation under the irradiation of light at 25 °C for 3 days. Then, cells were collected by centrifugation, and the collected cells were washed with the same volume of 50 mM phosphoric acid buffer solution (pH 7.7) as the culture solution. Thereafter, the obtained cells were suspended in the 200 ml of the buffer solution, thereby preparing washed cells. The washed cells obtained had a cell concentration of 32 g/L in terms of dry cell weight.

### 2. Sterilization treatment

To the washed cells, 1.5 % benzethonium chloride solution was added so as to have the concentrations as shown in Table 5, and sterilization treatment was conducted at 30 °C for 5 hours. Incidentally, for comparison, instead of 1.5 % benzethonium chloride solution, phosphoric acid buffer solution was added, and the same sterilization treatment was conducted.

### 3. Determination of viable cell count

After the treatment, the treated cells were washed three times with the buffer solution which had been used for the preparation of the washed cells, and suspended with the same buffer solution so that the volume became equal to one at sterilization treatment. Then, 0.1 ml of each suspension was applied on MYK agar medium and subjected to cultivation at 30 °C for 3 days, then obtaining viable cell count.

### 4. Determination of enzyme activity

With respect to nitrile hydratase activity, after 1.0 ml of 1M acrylic nitrile, 0.5 ml of 0.1M potassium phosphate buffer solution (pH 7.0) and 2 ml of the reaction mixture containing the washed cells to have been subjected to sterilizing treatment, were reacted with each other at 10°C for a predetermined period, 0.2 ml of IN HCl was added for stopping the reaction, and thereafter the amount of acrylamide generated was analized by gas chromatography for determination of the nitrile hydratase activity.

### (3) Rhodococcus sp. SK92 (FERM BP-3324)

### 1. Cultivation

Prepared was a medium (pH 7.2) comprising 20 g/L of sucrose, 5 g/L of polypepton, 1 g/L of KH₂PO₄, 1 g/L of K₂HPO₄, and 1 g/L of MgSO₄·7H₂O. 100 ml of the medium was dispensed into a 500 ml Erlenmeyer flask and sterilized by an autoclave at 120°C for 15 minutes. Rhodococcus sp. SK92 (FERM BP-3324) was inoculated into this medium and subjected to shaking cultivation at 30°C for 3 days. 1. ml of the thus obtained culture solution was inoculated into the same medium (20 Erlenmeyer flasks), to which simultaneously 2 ml of ethylene cyanohydrin was added, and subjected to shaking cultivation at 30°C for 20 hours. Thereafter, 3 ml of ethylene cyanohydrin were added and subjected to further cultivation for 3 days.

Then, cells were collected by centrifugation, and the collected cells were washed with the same volume of 50 mM phosphoric acid buffer solution (pH 7.7) as the culture solution. Thereafter, the obtained cells were suspended in 200 ml of the buffer solution, thereby preparing washed cells. The washed cells obtained had a cell concentration of 36 g/L in terms of dry cell weight.

### 2. Sterilization treatment

To the washed cells, 1.5 % benzethonium chloride solution was added so as to have the concentrations as shown in Table 5, and sterilization treatment was conducted at 30 °C for 5 hours. Incidentally, for comparison, instead of 1.5 % benzethonium chloride solution, phosphoric acid buffer solution was added, and the same sterilization treatment was conducted.

### 3. Determination of viable cell count

After the treatment, the cells were washed three times with the buffer solution which had been used for the preparation of the washed cells, and suspended with the same buffer solution so that the volume became equal to one at sterilization treatment. Then, 0.1 ml of each suspension was applied on MYK-Km agar medium and subjected to cultivation at 30 °C for 3 days, then obtaining viable cell count.

### 4. Determination of enzyme activity

With respect to nitrilase activity, after 2 ml of the reaction mixture containing 1.0 ml of 1M acrylic nitrile, 0.5 ml of 0.1 M potassium phosphate buffer solution (pH 7.0) and the washed cells to have been subjected to steriliziation treatment, were reacted at 10 °C for the predetermined period, 0.2 ml of IN HCl was added for stopping the reaction, and thereafter the amount of acrylic acid produced was analyzed by liquid chromatography for determination of the nitrilase activity.

### (4) Rhodococcus sp. EA4 (FERM BP-6231)

### 1. Cultivation

Prepared was a medium (pH 7.0) comprising 5 g/L of glycerol, 0.02 g/L of yeast extract, 0.5 g/L of MgSO₄·7H₂O, 1 g/L of KH₂PO₄, 1 g/L of K₂HPO₄. 100 ml of the medium was dispensed into a 500 ml Erienmeyer flask and sterilized by an autoclave at 120 °C for 15 minutes. *Rhodococcus sp.* EA4 (FERM BP-6231) was inoculated into this medium and subjected to a shaking cultivation at 30 °C for 3 days. 1 ml of the thus obtained culture solution was inoculated into the same medium (20 Erlenmeyer flasks), to simultaneously 5 g/L of acetamide was added, and subjected to a shaking cultivation at 30 °C for 48 hours.

Then, cells were collected by centrifugation, and the collected cells were washed with the same volume of 50 mM phosphoric acid buffer solution (pH 7.7) as the culture solution. Thereafter, the obtained cells were suspended in the 200 ml of the buffer solution, thereby preparing washed cells. The washed cells obtained had a cell concentration of 36 g/L in terms of dry cell weight.

### 2. Sterilization treatment

To the washed cells, 1.5 % benzethonium chloride solution was added so as to having the concentrations as shown in Table 5, and sterilization treatment was conducted at 30 °C for 5 hours. Incidentally, for comparison, instead of 1.5 % benzethonium chloride solution, phosphoric acid buffer solution was added, and the same sterilization treatment was conducted.

### 3. Determination of viable cell count

After the treatment, the cells were washed three times with the buffer solution which had been used for the preparation of the washed cells, and suspended with the same buffer solution so that the volume became equal to one at sterilization treatment. Then, 0.1 ml of each suspension was applied on MYK agar medium and subjected to cultivation at 30 °C for 3 days, then obtaining viable cell count.

### 4. Determination of enzyme activity

With respect to amidase activity, after 1 ml of the reaction mixture containing 0.5 ml of 0.05 M potassium phosphate buffer solution (pH 7.7) containing 0.2 M glycinamide and the washed cells to have been subjected to sterilization treatment, were reacted at 30 °C for a predetermined period, the cells were removed by centrifugation. Thereafter, the amount of glycine produced was analyzed by liquid chromatography for determination of the amidase activity.

### (5) R. rhodochrous ATCC12674/pAR016

### 1. Cultivation

*Rhodococcus rhodochrous* ATCC12674/pAR016 was inoculated into 100 ml MYK-Km medium and subjected to cultivation at 30 °C for 72 hours. A main cultivation was conducted with 5 L of the same medium (every 100 ml of the medium was each dispensed into a 500 ml Erlenmeyer flask), 1 % of the above pre-culture was inoculated and subjected to cultivation at 30 °C for 96 hours. Then, cells were collected by centrifugation, and the collected cells were washed with the same volume of 100 mM phosphoric acid buffer solution (pH 8.0) as the culture solution. Thereafter, the obtained cells were suspended in the 500 ml of the buffer solution, therety preparing washed cells. The washed cells obtained had a cell concentration of 42 g/L in terms of dry cell weight.

### 2. Sterilization treatment

To the washed cells, 1.5 % benzethonium chloride solution was added so as to have the concentrations as shown in Table 5, and sterilization treatment was conducted at 30 °C for 5 hours. Incidentally, for comparison, instead of 1.5 % benzethonium chloride solution, phosphoric acid buffer solution was added, and the same sterilization treatment was conducted.

### 3. Determination of viable cell count

After the treatment, the treated cells were washed three times with 100 mM phosphoric acid buffer solution (pH 8.0) containing 1 mM MgCl₂, and suspended with the same buffer solution so that the volume became equal to one at sterilization treatment.

After the above treatment, the treated cells were washed three times with the buffer solution which had been used for the preparation of the washed cells, and suspended with the same buffer solution so that the volume became equal to one at sterilization treatment. Then, 0.1 ml of each suspension was applied on MYK-Km agar medium and subjected to cultivation at 30 °C for 3 days, then obtaining viable cell count.

### 4. Determination of enzyme activity

With respect to aspartase activity, after 1 ml of the reaction mixture containing 0.5 ml of 0.05 M potassium phosphate buffer solution (pH 8.0) containing 1 M fumaric ammonium and 1mM MgCl₂ and the washed cells to have.been subjected to sterilization treatment, were reacted at 30 °C for a predetermined period, the cells were removed by centrifugation. Thereafter, the amount of asparatic acid produced was analyzed by liquid chromatography for determination of the aspartase activity.

### (6) Rhodococcus rhodochrous ATCC 12674

### 1. Cultivation

Prepared was a medium (pH 7.0) comprising 5 g/L of glucose, 0.02 g/L of yeast extract, 0.5 g/L of MgSP₄·7H₂O, 1 g/L of KH₂PO₄, and 1 g/L of K₂HPO₄. 100 ml of the medium was dispensed into a 500 ml Erlenmeyer flask and sterilized by an autoclave at 120 °C for 15 minutes. *Rhodococcus rhodochrous* ATCC12674 was inoculated into this medium and subjected to a shaking cultivation at 30 °C for 3 days. 1 ml of the thus obtained culture solution was inoculated into the same medium (20 Erlenmeyer flasks) further containing 10 g/L of fumaric acid, and subjected to shaking cultivation at 30 °C for 48 hours. Then, cells were collected by centrifugation, and the collected cells were washed with the same volume of 50 mM phosphoric acid buffer solution (pH 7.7) as the culture solution. Thereafter, the obtained cells were suspended in the 200 ml of the buffer solution, thereby preparing washed cells. The washed cells obtained had a cell concentration of 31 g/L in terms of dry cell weight.

### 2. Sterilization treatment

To the washed cells, 1.5 % benzethonium chloride solution was added so as to have the concentrations as shown in Table 5, and sterilization treatment was conducted at 30 °C for 5 hours. Incidentally, for comparison, instead of 1.5 % benzethonium chloride solution, phosphoric acid buffer solution was added, and the same sterilization treatment was conducted.

### 3. Determination of viable cell count

After the treatment, the treated cells were washed three times with 100 mM phosphoric acid buffer solution (pH 8.0) containing 1 mM MgCl₂, and suspended with the same buffer solution so that the volume became equal to one at sterilization treatment.

After the above treatment, the treated cells were washed three times with the buffer solution which had been used for the preparation of the washed cells, and suspended with the same buffer solution so that the volume became equal to one at sterilization treatment. Then, 0.1 ml of each suspension was applied on MYK agar medium and subjected to cultivation at 30 °C for 3 days, then obtaining viable cell count.

### 4. Determination of enzyme activity

With respect to fumarase activity, after 1 ml of the reaction mixture containing 0.5 ml of 0.05 M potassium phosphate buffer solution (pH 7.7) containing 0.2 M fumaric acid and the washed cells to have been subjected to sterilization treatment, were reacted at 30 °C for a predetermined period, the cells were removed by centrifugation, and thereafter the amount of malic acid produced was analyzed by liquid chromatography for determination of the fumarase activity.

**Table 5**

| | Examined microbial cell | Enzyme | Treatment condition | Viable cell count (cells/ml) | Remaining act ivity (%) |
|---|---|---|---|---|---|
| 1 | J-1 | nitrile hydratase | phosphoric acid buffer solution | >10⁵ | 100 |
| 2 | J-1 | nitrile hydratase | 1.5% benzethonium chloride | 0 | 95 |
| 3 | J-1 | nitrile hydratase | 1.5% benzethonium chloride | 25 | 97 |
| 4 | J-1 | nitrile hydratase | 0.25% glutaraldehyde | 1200 | 98 |
| 5 | J-1 | nitrile hydratase | 0.2% benzethonium chloride+ 0.25% glutaraldehyde | 0 | 98 |
| 6 | ATCC12674/pKNH2 | nitrile hydratase | phosphoric acid buffer solution. | >10⁵ | 120 |
| 7 | ATCC12674/pKNH2 | nitrile hydratase | 0.25% benzethonium chloride | 0 | 94 |
| 8 | SK-92 | nitrilase | phosphoric acid buffer solution. | >10⁵ | 98 |
| 9 | AK-92 | nitrilase | 0,25% benzethonium chloride | 0 | 84 |
| 10 | EA-4 | amidase | phosphoric acid buffer solution. | >10⁵ | 99 |
| 11 | EA-4 | amidase | 0.25% benzethonium chloride | 0 | 91 |
| 12 | ATCC12674/pAR016 | aspartase | phosphoric acid buffer solution. | >10⁵ | 100 |
| 13 | ATCC12674/pAR016 | aspartase | 0.25% benzethonium chloride | 0 | 94 |
| 14 | ATCC12674 | fumarase | phosphoric acid buffer solution. | >10⁵ | 100 |
| 15 | ATCC12674 | fumarase | 0.25% benzethonium chloride | 0 | 98 |

### INDUSTRIAL APPLICABILITY

Accroding to the present invention, sterilized microbial cells can be produced without deactivating an industrially useful enzyme or the like, and therefore this permits obviating a secondary contamination by leaking viable cells when microbial cells are industrially used. In particularly, when gene-recombinant cells are used, performance of sterilization treatment according to the present invention facilitates handling the microbial cells in the later process and does not require a special facility. Thus, the present invention can provide an industrially useful means.

## Claims

1. A process for the sterilisation of a bacterium without deactivation of enzymatic activity, comprising the addition of
(i) a surfactant
(ii) glutaraldehyde
wherein the surfactant and glutaraldehyde are added to the bacterium so as to have concentrations of 0.05 to 10% and 0.05 to 5%, respectively, and
wherein the bacterium has a dry cell weight of 5 to 200 g/L in an aqueous medium,
wherein the bacterium has at least one enzyme activity selected from the group consisting of nitrile hydratase, nitrilase, amidase, fumarase, aspartase, and ethylenediamine-N, N'-disuccinic acid: ethylenediamine lyase; and
wherein the surfactant is a cationic surfactant or an ampholytic surfactant, and
wherein the bacterium is selected from a gene-recombinant bacterium, Rhodococcus rhodochrous having the deposit number FERM BP-1478, Rhodococcus rhodochrous having the deposit number FERM BP-3733, Rhodococcus sp. having the deposit number FERM BP-3324, Rhodococcus sp. having the deposit number FERM-BP-6231, Bacillus subtilis having the deposit number ATCC 6051, Bacillus stearothermophilus having the deposit number ATCC 12016, Rhodococcus rhodochrous having the deposit number ATCC 12674, Rhodococcus rhodochrous ATCC 12674/pAR016 as described in JP 10-337185, and Rhodococcus rhodochrous having the deposit number FERM BP-6548.

2. The process according to claim 1, wherein the cationic surfactant is at least one member selected from the group consisting of benzethonium chloride, cetylpyridinium chloride, methylstearoyl chloride, and cetyltrymethylammonium bromide.

3. The process according to claim 1, wherein the bacterium is a gene recombinant bacterium.

4. The process according to claim 3, wherein the gene recombinant bacterium is a genus Rhodococcus bacterium.

5. A solution containing a sterilised bacterium, wherein the bacterium has a dried cell weight of 5 to 200 g/L in an aqueous medium, comprising
(i) a surfactant having a concentration of 0.05 to 10%,
(ii) glutaraldehyde having a concentration of 0.05 to 5%,
wherein the bacterium has at least one enzyme activity selected from the group consisting of nitrile hydratase, nitrilase, amidase, fumarase, aspartase, and ethylenediamine-N, N'-disuccinic acid: ethylenediamine lyase, wherein the enzymatic activity is not deactivated, and wherein the surfactant is a cationic surfactant or an ampholytic surfactant, and wherein the bacterium is selected from a gene-recombinant bacterium, Rhodococcus rhodochrous having the deposit number FERM BP-1478, Rhodococcus rhodochrous having the deposit number FERM BP-3733, Rhodococcus sp. having the deposit number FERM BP-3324, Rhodococcus sp. having the deposit number FERM-BP-6231, Bacillus subtilis having the deposit number ATCC 6051, Bacillus stearothermophilus having the deposit number ATCC 12016, Rhodococcus rhodochrous having the deposit number ATCC 12674, Rhodococcus rhodochrous ATCC 12674/pAR016 as described in JP 10-337185, and Rhodococcus rhodochrous having the deposit number FERM BP-6548.

6. The solution according to claim 5, wherein the cationic surfactant is at least one member selected from the group consisting of benzethonium chloride, cetylpyridinium chloride, methylstearoyl chloride, and cetyltrymethylammonium bromide.

7. The solution according to claim 6, wherein the concentration of benzethonium chloride is 0.05 to 5%.

8. The solution according to claim 5, wherein the gene-recombinant bacterium is a genus Rhodococcus bacterium.

## Patentansprüche

1. Verfahren zur Sterilisation eines Bakteriums ohne Inaktivierung der Enzymaktivität, das das Hinzufügen von
(i) einem Tensid
(ii) Glutaraldehyd
umfasst,
wobei das Tensid und Glutaraldehyd zu dem Bakterium hinzugefügt werden, so dass sie Konzentrationen von 0,05 bis 10% beziehungsweise 0,05 bis 5% haben und
wobei das Bakterium ein Trockenzellgewicht von 5 bis 200 g/l in einem wässrigen Medium besitzt,
wobei das Bakterium wenigstens eine Enzymaktivität, ausgewählt aus der Gruppe bestehend aus Nitrilhydratase, Nitrilase, Amidase, Fumarase, Aspartase und Ethylendiamin-N,N'-dibernsteinsäure:Ethylendiaminlyase, besitzt; und wobei das Tensid ein kationisches oder ein ampholytisches Tensid ist und
wobei das Bakterium ausgewählt ist aus einem genrekombinanten Bakterium, *Rhodococcus rhodochrous* mit der Hinterlegungsnummer FERM BP-1478, *Rhodococcus rhodochrous* mit der Hinterlegungsnummer FERM BP-3733, *Rhodococcus sp.* mit der Hinterlegungsnummer FERM BP-3324, *Rhodococcus sp.* mit der Hinterlegungsnummer FERM BP-6231, *Bacillus subtilis* mit der Hinterlegungsnummer ATCC 6051, *Bacillus stearothermophilus* mit der Hinterlegungsnummer ATCC 12016, *Rhodococcus rhodochrous* mit der Hinterlegungsnummer ATCC 12674, *Rhodococcus rhodochrous* ATCC 12674/pAR016 wie in JP 10-337185 beschrieben und *Rhodococcus rhodochrous* mit der Hinterlegungsnummer FERM BP-6548.

2. Verfahren gemäß Anspruch 1, wobei das kationische Tensid mindestens ein Mitglied ist, ausgewählt aus der Gruppe bestehend aus Benzethoniumchlorid, Cetylpyridiniumchlorid, Methylstearoylchlorid und Cetyltrimethylammoniumbromid.

3. Verfahren gemäß Anspruch 1, wobei das Bakterium ein genrekombinantes Bakterium ist.

4. Verfahren gemäß Anspruch 3, wobei das genrekombinante Bakterium ein Bakterium der Gattung *Rhodococcus* ist.

5. Lösung, die ein sterilisiertes Bakterium enthält, wobei das Bakterium ein Trockenzellgewicht von 5 bis 200 g/l in einem wässrigen Medium besitzt, die umfasst
(i) ein Tensid, das eine Konzentration von 0,05 bis 10% besitzt,
(ii) Glutaraldehyd, das eine Konzentration von 0,05 bis 5% besitzt, wobei das Bakterium mindestens eine Enzymaktivität ausgewählt aus der Gruppe bestehend aus Nitrilhydratase, Nitrilase, Amidase, Fumarase, Aspartase und Ethylendiamin-N,N'-dibernsteinsäure:Ethylendiaminlyase besitzt, wobei die Enzymaktivität nicht inaktiviert wird und wobei das Tensid ein kationisches oder ein ampholytisches Tensid ist und wobei das Bakterium ausgewählt ist aus einem genrekombinanten Bakterium, *Rhodococcus rhodochrous* mit der Hinterlegungsnummer FERM BP-1478, *Rhodococcus rhodochrous* mit der Hinterlegungsnummer FERM BP-3733, *Rhodococcus sp.* mit der Hinterlegungsnummer FERM BP-3324, *Rhodococcus sp.* mit der Hinterlegungsnummer FERM BP-6231, *Bacillus subtilis* mit der Hinterlegungsnummer ATCC 6051, *Bacillus stearothermophilus* mit der Hinterlegungsnummer ATCC 12016, *Rhodococcus rhodochrous* mit der Hinterlegungsnummer ATCC 12674, *Rhodococcus rhodochrous* ATCC 12674/pAR016 wie in JP 10-337185 beschrieben und *Rhodococcus rhodochrous* mit der Hinterlegungsnummer FERM BP-6548.

6. Lösung gemäß Anspruch 5, wobei das kationische Tensid mindestens ein Mitglied ist, ausgewählt aus der Gruppe bestehend aus Benzethoniumchlorid, Cetylpyridiniumchlorid, Methylstearoylchlorid und Cetyltrimethylammoniumbromid.

7. Lösung gemäß Anspruch 6, wobei die Konzentration von Benzethoniumchlorid 0,05 bis 5% ist.

8. Lösung gemäß Anspruch 5, wobei das genrekombinante Bakterium ein Bakterium der Gattung *Rhodococcus* ist.

## Revendications

1. Processus pour la stérilisation d'une bactérie, sans désactivation de l'activité enzymatique, comprenant le fait d'ajouter
(i) un agent de surface
(ii) du glutaraldéhyde
dans lequel l'agent de surface et le glutaraldéhyde sont ajoutés à la bactérie de manière à avoir des concentrations allant de 0,05 à 10% et allant de 0,05 à 5%, respectivement, et
dans lequel la bactérie a un poids cellulaire sec allant de 5 à 200 g/L dans un milieu aqueux,
dans lequel la bactérie a au moins une activité enzymatique choisie dans le groupe constitué de la nitrile hydratase, de la nitrilase, de l'amidase, de la fumarase, de l'aspartase et de l'acide éthylénediamine-N, N'-disuccinique : éthylènediamine lyase ; et
dans lequel l'agent de surface est un agent de surface cationique ou un agent de surface ampholyte, et
dans lequel la bactérie est choisie parmi une bactérie à recombinaison génique, *Rhodococcus rhodochrous* ayant le numéro de dépôt FERM BP-1478, *Rhodococcus rhodochrous* ayant le numéro de dépôt FERM BP-3733, *Rhodococcus* sp. ayant le numéro de dépôt FERM BP-3324, *Rhodococcus* sp. ayant le numéro de dépôt FERM-BP-6231, *Bacillus subtilis* ayant le numéro de dépôt ATCC 6051, *Bacillus stearothermophilus* ayant le numéro de dépôt ATCC 12016, *Rhodococcus rhodochrous* ayant le numéro de dépôt ATCC 12674, *Rhodococcus rhodochrous* ATCC 12674/pAR016 telle que décrite dans le document JP 10-337185 et *Rhodococcus rhodochrous* ayant le numéro de dépôt FERM BP-6548.

2. Processus selon la revendication 1, dans lequel l'agent de surface cationique est au moins un élément choisi dans le groupe constitué du chlorure de benzéthonium, du chlorure de cétylpyridinium, du chlorure de méthylstéaroyl et du bromure de cétyltryméthylammonium.

3. Processus selon la revendication 1, dans lequel la bactérie est une bactérie à recombinaison génique.

4. Processus selon la revendication 3, dans lequel la bactérie à recombinaison génique est une bactérie du genre *Rhodococcus.*

5. Solution contenant une bactérie stérilisée, dans laquelle la bactérie a un poids cellulaire sec allant de 5 à 200 g/L dans un milieu aqueux, comprenant
(i) un agent de surface ayant une concentration allant de 0,05 à 10%,
(ii) un glutaraldéhyde ayant une concentration allant de 0,05 à 5%,
dans laquelle la bactérie a au moins une activité enzymatique choisie dans le groupe constitué de la nitrile hydratase, de la nitrilase, de l'amidase, de la fumarase, de l'aspartase et de l'acide éthylénediamine-N, N'-disuccinique éthylènediamine lyase, dans laquelle l'activité enzymatique n'est pas désactivée, et dans laquelle l'agent de surface est un agent de surface cationique ou un agent de surface ampholyte, et dans laquelle la bactérie est choisie parmi une bactérie à recombinaison génique, *Rhodococcus rhodochrous* ayant le numéro de dépôt FERM BP-1478, *Rhodococcus rhodochrous* ayant le numéro de dépôt FERM BP-3733, *Rhodococcus* sp. ayant le numéro de dépôt FERM BP-3324, *Rhodococcus* sp. ayant le numéro de dépôt FERM BP-6231, *Bacillus subtilis* ayant le numéro de dépôt ATCC 6051, *Bacillus stearothermophilus* ayant le numéro de dépôt ATCC 12016, *Rhodococcus rhodochrous* ayant le numéro de dépôt ATCC 12674, *Rhodococcus rhodochrous* ATCC 12674/pAR016 telle que décrite dans le document JP 10-337185 et *Rhodococcus rhodochrous* ayant le numéro de dépôt FERM BP-6548.

6. Solution selon la revendication 5, dans laquelle l'agent de surface cationique est au moins un élément choisi dans le groupe constitué du chlorure de benzéthonium, du chlorure de cétylpyridinium, du chlorure de méthylstéaroyl et du bromure de cétyltryméthylammonium.

7. Solution selon la revendication 6, dans laquelle la concentration de chlorure de benzéthonium est de 0,05 à 5%.

8. Solution selon la revendication 5, dans laquelle la bactérie à recombinaison génique est une bactérie du genre *Rhodococcus.*
